# EUROPÄISCHE PATENTSCHRIFT

(11) **EP 2 855 031 B1**
(45) Veröffentlichungstag und Bekanntmachung des Hinweises auf die Patenterteilung: **28.08.2019**
(21) Anmeldenummer: 13730133.9
(22) Anmeldetag: 24.05.2013
(51) Int. Cl.: B05D 1/06, B05D 1/12, B05D 1/26, H01B 3/46, B05D 5/08, B05D 7/02, B05D 7/20, H01B 13/16, C08L 83/00, A61L 31/14, A61N 1/05, H01B 13/14

(54) **VERFAHREN ZUR HERSTELLUNG EINES ELEMENTS, INSBESONDERE EINES KABELS, AUS EINEM SILIKONARTIGEN GRUNDMATERIAL UMFASSEND DIE EINBRINGUNG VON FESTSTOFFPARTIKELN IN DIE OBERFLÄCHE EINES ZWISCHENPRODUKTS**
METHOD OF MANUFACTURING AN ELEMENT, IN PARTICULAR A CABLE, FROM A SILICONE-LIKE BASE MATERIAL INCLUDING INTEGRATING SOLID PARTICLES INTO THE SURFACE OF AN INTERMEDIATE PRODUCT
PROCÉDÉ POUR LA FABRICATION D'UN ÉLÉMENT, EN PARTICULIER D'UN CÂBLE, À PARTIR D'UN MATÉRIAU DE TYPE SILICONE COMPRENANT L'INTÉGRATION DE PARTICULES SOLIDES DANS LA SURFACE D'UN PRODUIT INTERMÉDIAIRE

(30) Priorität: 25.05.2012 DE 102012208871
(43) Veröffentlichungstag der Anmeldung: 08.04.2015
(73) Patentinhaber: LEONI Kabel GmbH, 90402 Nürnberg (DE)
(72) Erfinder: DREINER, Michael, 51688 Wipperfürth (DE); NORDING, Albert, 26219 Bösel (DE)
(74) Vertreter: FDST Patentanwälte
(86) Internationale Anmeldenummer: PCT/EP2013/060807
(87) Internationale Veröffentlichungsnummer: WO 2013/175012

(56) Entgegenhaltungen:
- EP-B1- 1 691 374
- CH-A5- 605 127
- US-A- 5 650 193
- US-A1- 2003 095 762
- US-A1- 2010 075 018

## Beschreibung

Die Erfindung betrifft ein Verfahren zur Herstellung eines Elements, insbesondere eines Kabels, welches außenseitig ein silikonartiges Grundmaterial aufweist. Die Erfindung betrifft weiterhin ein derartiges Element.

Bei dem Element handelt es sich insbesondere um ein elektrisches Kabel mit einem Kabelmantel aus Silikon. Die Erfindung ist jedoch nicht auf das Anwendungsgebiet von elektrischen Kabeln beschränkt.

Elektrische Kabel mit einem Silikonmantel werden beispielsweise in der Medizintechnik aufgrund ihrer besonderen Eigenschaften eingesetzt. Zum einen zeichnet sich der Werkstoff Silikon nämlich durch eine sehr große Flexibilität oder Elastizität aus, so dass dadurch sehr flexible und elastische Kabel beispielsweise im Bereich von 1mm bis 10mm Durchmesser ausgebildet werden können. Gleichzeitig weist das Silikon auch eine hohe Dauergebrauchstemperatur von etwa 180°C auf. Hierdurch sind derartige Kabel für medizinische Anwendungen geeignet, bei denen die Kabel regelmäßig sterilisiert werden müssen. Dies erfolgt üblicherweise mit Hilfe einer sogenannten Dampfsterilisation bei Temperaturen von etwa 140°C bis 150°C.

Aus der EP 2 409 725 A1 ist eine Elektrodenleitung für den medizinischen Anwendungsbereich zu entnehmen, bei der in einen Isolationsschlauch elektrische Leiter eingeführt sind, die im Schlauch daher in gewisser Weise lose einliegen. Die Elektrodenleitung dient zur intrakorporalen Anwendung beispielsweise für Herzschrittmacher-Elektrodenleitungen. Zur Verbesserung der Abriebfestigkeit des Schlauches ist in den Grundwerkstoff des Schlauches partikelförmiges Füllmaterial eingebracht. Bei dem Grundmaterial handelt es sich beispielsweise um Silikon, Polyurethan, Polyamid, PTFE etc. Zur Herstellung des Isolationsschlauches werden die Füllpartikel einem Extrudat beigemengt und anschließend zum Isolationsschlauch extrudiert oder gespritzt. In einer Ausführungsvariante wird mittels Koextrusion ein zweischichtiger Isolationsschlauch ausgebildet, bei dem nur die äußere Schicht mit dem Füllmaterial angefüllt ist. Alternativ zur Extrusion wird ein Grundschlauch in einen dünnflüssigen Grundwerkstoff getaucht, welcher mit dem Filmmaterial versetzt ist, um die äußere Schicht auszubilden.

Aufgrund der guten Temperaturbeständigkeit von Silikon, wird Silikon insbesondere auch bei feuerresistenten Kabeln herangezogen. Zum Verbessern der Feuerwiderstandsfähigkeit ist gemäß der JP 2001035267 ebenfalls das Einbringen von Füllwerkstoffen, nämlich Glimmer-Partikel, vorgesehen.

Glimmer-Partikel werden beispielsweise gemäß der CH 605 127 A5 auch für eine Schutzbeschichtung von Kunststoff-Profilteilen zur Erhöhung der Brandresistenz eingesetzt.

Aus der EP 1 691 374 B1 ist ein Kabel für die Medizintechnik zu entnehmen, bei dem der Kabelmantel aus einem Silikonkautschuk besteht, wobei zur Verringerung der Klebrigkeit des Kabelmantels in diesen von außen her Metallseifen integriert sind.

Aus der US 2003/0095762 A1 ist ein optisches Datenkabel mit optischen Fasern zu entnehmen, bei dem die einzelnen optischen Fasern eine äußere Schicht aufweisen, die Mikroglaskugeln enthält, um eine geringe Reibung zwischen dem optischen Faserkabel und einer Umhüllung zu erreichen.

Ausgehend hiervon liegt der Erfindung die Aufgabe zugrunde, ein Verfahren zur Herstellung eines Elements, insbesondere eines Kabels sowie ein vorzugsweise nach diesem Verfahren hergestelltes Element, insbesondere Kabel anzugeben, welches sich insbesondere für den medizinischen Bereich eignet und gegenüber den bekannten Kabeln verbesserte Eigenschaften bei einfacher Herstellung erlaubt.

Die Aufgabe wird gemäß der Erfindung gelöst durch ein Verfahren zur Herstellung eines Elements, insbesondere eines Kabels, das außenseitig ein silikonartiges Grundmaterial aufweist. Das silikonartige Grundmaterial wird in einem ersten Schritt zunächst zu einem unvernetzten, zumindest nicht vollständig vernetzten Zwischenprodukt ausgebildet. Anschließend werden in einem zweiten Schritt Feststoffpartikel in die Oberfläche eingebracht und schließlich wird in einem dritten Schritt das unvernetzte oder nur teilweise vernetzte Grundmaterial bis zum gewünschten Vernetzungsgrad vernetzt, insbesondere vollständig vernetzt.

Bei diesem Verfahren wird daher der zumindest teilweise unvernetzte Zustand des Grundmaterials zum Einbringen der Feststoffpartikel in die Oberfläche des Grundmaterials ausgenutzt. Aufgrund des unvernetzten Zustands ist dies prozesstechnisch in einfacher Weise möglich. Durch den sich anschließenden Vernetzungsvorgang werden dann die Feststoffpartikel fest in die Oberfläche eingebunden. Die Feststoffpartikel werden als solche lose aufgebracht, sind daher nicht in einem Trägermaterial eingebettet. Die Feststoffpartikel liegen nach Art eines Pulvers vor.

Die Feststoffpartikel sind daher ausschließlich in die Oberfläche von außen eingebracht. Es erfolgt keine Einbindung der Feststoffpartikel als Füllmaterial in das Volumen des Grundmaterials. Aufgrund des gewählten Herstellverfahrens ist die Eindringtiefe der Feststoffpartikel daher in der Regel auch maximal auf die Ausdehnung der Feststoffpartikel begrenzt. Die Feststoffpartikel werden daher dem Grundmaterial beim Herstellungsprozess nicht beigemengt.

Dieses Verfahren beruht auf der Erkenntnis, dass Elemente, insbesondere Kabel mit einem außenseitigen Material, welches zumindest silikonartig ist oder aus Silikon besteht, einen hohen Reibungskoeffizienten haben. Dies führt oftmals zu einem regelrechten "Anhaften" des Elements an Oberflächen, wie beispielsweise an einem Operationstisch oder auch auf der menschlichen Haut.

Ausgehend hiervon liegt der Erfindung die Überlegung zugrunde, die silikonspezifische Haptik durch das Einbringung von Feststoffpartikeln ausschließlich an der Oberfläche günstig zu beeinflussen und insbesondere den Reibungskoeffizienten an der Oberfläche deutlich zu vermindern, um das unerwünschte Anhaften zu vermeiden oder zumindest zu reduzieren. Durch das Anbringen der Feststoffpartikel lediglich auf der Oberfläche stehen die Feststoffpartikel regelmäßig etwas über die durch das Grundmaterial gebildete Oberfläche über. Die Reibung wird daher maßgeblich durch die Feststoffpartikel bestimmt. Aufgrund deren Beschaffenheit und Struktur im Vergleich zu Silikon ist die Reibung deutlich vermindert. Vorteilhaft wirkt sich darüber hinaus die lose Verteilung der Feststoffpartikel über die Oberfläche aus, so dass keine vollständige Flächenreibung sondern jeweils nur eine punktuelle Kontaktzone auftritt.

Unter silikonartigem Grundmaterial werden allgemein vernetzungsfähige Kunststoff-Materialien verstanden, welche im vernetzten Endzustand insbesondere einen zu Silikon vergleichbaren Reibungskoeffizienten aufweisen. Weitere Grundmaterialien sind neben Silikonen auch Polyurethane, Polyamide, Polytetrafluorethylen (PTFE), Ethylen-Tetrafluorethylen (ETFE), Perfluorethylenpropylen (FEP), Perfluoralkoxy (PFA) etc. sowie Copolymere hiervon. Vorzugsweise wird jedoch für das Grundmaterial Silikon oder ein silikonenthaltenes Material (Copolymer) verwendet.

Die Feststoffpartikel werden auf mechanischem Weg in Oberfläche des Zwischenprodukts eingebracht. Hierbei wird allgemein eine mechanische Kraft auf die Partikel ausgeübt. Im einfachsten Fall kann dies die Gewichtskraft sein. Alternativ oder als Unterstützung zu einem mechanischen Aufbringen besteht auch die Möglichkeit eines elektrostatischen Aufbringens der Feststoffpartikel. Hierbei werden anstelle von mechanischen Kräften elektro(statische) Kräfte ausgenutzt. Die lose vorliegenden Feststoffpartikel werden hierbei vor dem Aufbringen insbesondere elektrostatisch aufgeladen.

Für das mechanische Einbringen wird das Zwischenprodukt im zweiten Schritt durch einen Vorratsbehälter mit darin enthaltenen Feststoffpartikeln geführt. Da das Zwischenprodukt aufgrund seines zumindest teilweise unvernetzten Zustands üblicherweise eine hohe Klebfähigkeit aufweist, bleiben die Feststoffpartikel automatisch an der Oberfläche hängen.

Um dieses Anhaften der Feststoffpartikel zu unterstützen und um einen möglichst homogenen Deckungsgrad zu erzielen wird der Vorratsbehälter einer Schwingung ausgesetzt, so dass die Feststoffpartikel quasi in die Oberfläche mechanisch eingepresst werden. Der Vorratsbehälter ist daher als ein Rüttelbehälter ausgebildet. Grundsätzlich könnte auch das Element selbst mechanisch hin und her bewegt werden.

In zweckdienlicher Weiterbildung ist der Vorratsbehälter nach Art eines Trichters ausgebildet, durch den das Zwischenprodukt geführt wird. Dies erlaubt insbesondere auch ein vertikales Durchführen des insbesondere als Strang ausgebildeten Zwischenprodukts in einem Endlosprozess durch den Vorratsbehälter. Alternative Formen des Vorratsbehälters sind ebenfalls möglich.

Alternativ hierzu wird das Zwischenprodukt durch einen Wirbelstrombehälter geführt, in dem ein Luftstrom oder ein Wirbel als Trägerstrom für die Feststoffpartikel ausgebildet ist, so dass diese aufgewirbelt werden und dadurch am Mantel des Zwischenprodukts haften bleiben.

Das Zwischenprodukt selbst wird zweckdienlicherweise durch einen Extrusionsprozess ausgebildet, wobei vorzugsweise unmittelbar anschließend in einem kontinuierlichen Prozess die Feststoffpartikel eingebracht werden. Insbesondere wird hierzu das nach Art eines Endlos-Strangs ausgebildete Zwischenprodukt durch den Vorratsbehälter geführt.

In bevorzugter Weiterbildung werden überschüssige Feststoffpartikel in einer Reinigungsstufe wieder entfernt. Dies erfolgt insbesondere nach dem dritten Schritt des Vernetzens, so dass also alle nicht in die Oberfläche eingebundene Feststoffpartikel wieder entfernt werden. Dies erfolgt vorzugsweise durch einen Waschprozess. Hierzu wird das Element zweckdienlicherweise durch ein Reinigungsbad geführt, welches beispielsweise ergänzend mit Ultraschall beaufschlagt ist.

Als Feststoffpartikel werden Schichtsilikate (Glimmerteilchen) in die Oberfläche eingebracht. Untersuchungen haben gezeigt, dass der gewünschte Effekt der Reduzierung der Reibung bei Glimmerteilchen besonders markant ist. Auch Talkpartikel haben sich als geeignet herausgestellt.

Untersuchungen haben weiterhin gezeigt, dass die Größe der Feststoffpartikel einen entscheidenden Einfluss auf die gewünschte reibungsmindernde Eigenschaft hat. Die Feststoffpartikel weisen bevorzugt eine Größe auf, die einer Meshgröße im Bereich von 320 +/- 40 entspricht. Mesh ist dabei die Maschenweite eines Siebes zur Absonderung der gewünschten Partikel. Die Feststoffpartikel weisen daher vorzugsweise allgemein eine maximale Partikelgröße im Bereich vorzugsweise von 35 µm bis 55µm auf.

Im Hinblick auf ein besonders effizientes und ökonomisches Herstellverfahren wird das Element, insbesondere Kabel in einem kontinuierlichen, vollautomatischen Prozess hergestellt, wobei folgende Schritte unmittelbar aufeinander folgend ausgeführt werden: Zunächst wird das Zwischenprodukt als kontinuierlicher Strang vorzugsweise in einem Extrusionsprozess erzeugt, anschließend werden in die Oberfläche des extrudierten Stranges Feststoffpartikel eingebracht, bevor dann das Grundmaterial vernetzt wird. Anschließend wird das so erhaltene Produkt von überschüssigen Feststoffpartikeln gereinigt und schließlich wird das erhaltene Produkt typischerweise beispielsweise auf einer Trommel aufgewickelt.

Bei dem Element handelt es sich vorzugsweise allgemein um ein als Mantelleitung ausgebildetes elektrisches Kabel, mit zumindest einer Leitungsader, die von einem Außenmantel aus dem silikonartigen Grundmaterial umgeben ist. Bei dem Kabel handelt es sich insbesondere um ein Daten- oder Signalkabel, bei dem mehrere isolierte Leitungsadern von einem gemeinsamen Außenmantel umgeben sind. Die Leitungsadern weisen typischerweise einen elektrischen Leiter auf, der selbst von einer Isolierung umgeben ist. Bei dem elektrischen Leiter kann es sich um einen massiven Draht und vorzugsweise um eine feinst- bzw. feindrähtige Litze handeln. Das gesamte Kabel weist beispielsweise einen Durchmesser im Bereich von etwa 1mm - 5mm auf, wobei die Mantelstärke des Silikon-Außenmantels beispielsweise im Bereich von 0,5mm bis 3mm liegt.

Die Aufgabe wird weiterhin gemäß der Erfindung gelöst durch ein Element mit den Merkmalen des Anspruchs 11. Die im Hinblick auf das Herstellverfahren ausgeführten Vorteile in bevorzugten Ausgestaltungen sind sinngemäß auch auf das Element zu übertragen.

Dieses Element ist dadurch gekennzeichnet, dass lediglich in der Oberfläche des Grundmaterials Feststoffpartikel eingebettet sind. Die Feststoffpartikel liegen daher lediglich in einem oberflächennahen Bereich vor, das Volumen des Grundmaterials selbst ist daher frei von Feststoffpartikeln. Die Eindringtiefe der Partikel in die Oberfläche entspricht daher vorzugsweise lediglich maximal der Partikelgröße.

Weiterhin steht zumindest ein Teil der Feststoffpartikel schuppenartig aus der Oberfläche hervor. Die Feststoffpartikel weisen dabei vorzugsweise auch keinerlei Vorzugsorientierung auf, sondern sind wahllos in die Oberfläche eingebettet. Die Feststoffpartikel sind plättchenförmige Glimmerpartikel und weisen eine Größe vorzugsweise im Bereich von 35µm - 55µm auf (gemessen in der größten Ausdehnungsrichtung).

Ausführungseispiele der Erfindung werden nachfolgend anhand der Figuren näher erläutert. Diese zeigen jeweils in vereinfachten Darstellungen:
- FIG 1A-C: Schaubilder zur Illustration des Herstellverfahrens zur Herstellung eines Kabels mit einem Silikon-Außenmantel mit darin eingebrachten Feststoffpartikeln,
- FIG 2: einen stark vereinfachten Querschnitt durch ein danach hergestelltes Kabel sowie
- FIG 3: eine Mikroskopaufnahme der Oberfläche eines derartig hergestellten Kabels.

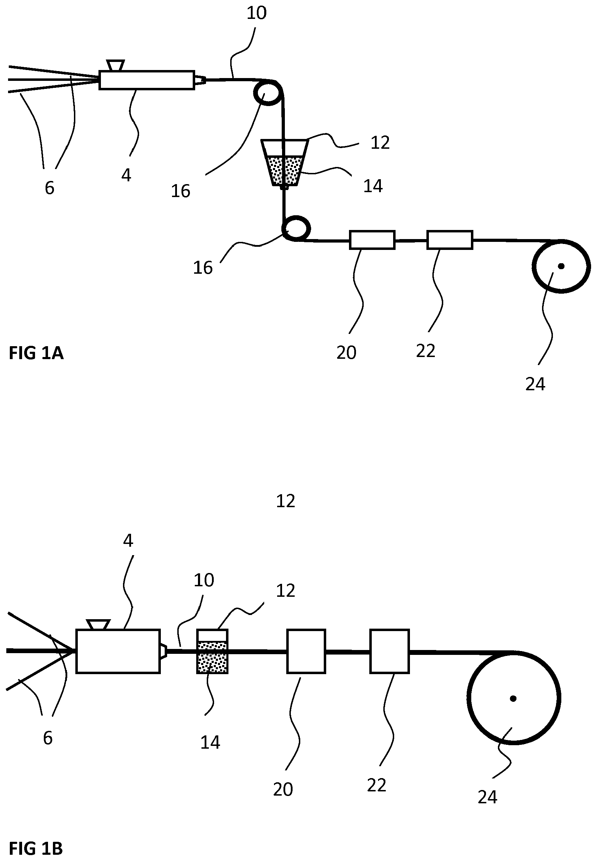

Zur Herstellung eines zunächst als Endlos-Ware ausgebildeten Kabels 2 können unterschiedliche Anlagen und Komponenten eingesetzt werden. Beispielhaft sind in den Figuren 1A bis 1C drei zueinander jeweils modifizierte Varianten dargestellt.

In allen Varianten werden einem Extruder 4 mehrere Leitungsadern 6 zugeführt und im Rahmen eines Extrusionsprozesses mit einem Außenmantel 8 aus einem silikonartigen Grundmaterial umgeben. Der Außenmantel umschließt dabei die Leitungsadern 8 unmittelbar. Er liegt daher an den Leitungsadern 6 unmittelbar an. Neben den Leitungsadern 6 sind bei Bedarf weiterhin Füll- oder Schlauchelemente oder auch Zugentlastungsfäden mit eingearbeitet.

Als Grundmaterial wird ein Silikon herangezogen, welches beim Verlassen des Extruders 4 noch in einem zumindest teilweise unvernetzten Zustand ist. Aus dem Extruder 4 tritt daher ein Zwischenprodukt 10 aus. Dieses wird in einem kontinuierlichen Prozess anschließend durch einen (Vorrats-) Behälter 12 hindurch geführt, welcher mit als loses Schüttgut vorliegenden Feststoffpartikeln 14, insbesondere Glimmerpartikel angefüllt ist oder in den zumindest die Feststoffpartikel eingebracht werden.

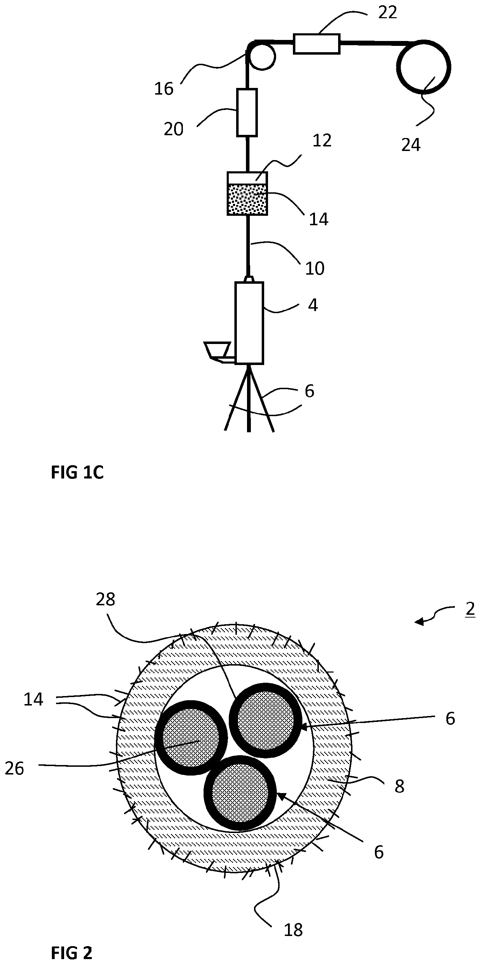

Bei dem Vorratsbehälter 12 handelt es sich gemäß der Ausführungsvariante nach Fig. 1A bevorzugt um einen Rütteltrichter, der in Schwingungen versetzbar ist. Das strangförmige Zwischenprodukt 10 durchläuft diesen Vorratsbehälter 12 vorzugsweise in vertikaler Richtung, wahlweise von oben nach unten (Fig. 1A) oder von unten nach oben (Fig. 1C). Alternativ ist eine horizontale Durchführung durch den Vorratsbehälter vorgesehen (Fig. 1B). Der Vorratsbehälter in Fig. 1B ist dabei vorzugsweise nach Art eines Wirbelstrombehälters ausgebildet, in dem ein Luftstrom erzeugt wird, so dass die vorzugsweise im Vorratsbehälter 12 bevorratete Feststoffpartikel 14 aufgewirbelt werden.

Schließlich werden in einer weiteren, nicht dargestellten Alternative die Feststoffpartikel 14 mit elektrostatischer Unterstützung aufgebracht. Hierzu werden die Feststoffpartikel 14 zunächst elektrostatisch aufgeladen und anschließend mit dem Zwischenprodukt 10 in Kontakt gebracht. Dies erfolgt vorzugsweise wiederum mit Hilfe eines Behälters 12 - ähnlich z.B. wie in Fig. 1B dargestellt - indem die Feststoffpartikel 14 durch ein elektrostatisches Aufladungsmodul hindurchgeführt und anschließend von oben nach Art einer Berieselungsanlage auf das Zwischenprodukt 10 aufgebracht werden. Bei Bedarf kann hierbei eine Unterstützung durch einen Luftstrom zur Erzeugung von Verwirbelungen erfolgen.

Nach dem Aufbringen der Feststoffpartikel 14 auf die Oberfläche 18 (vgl. FIG 2) wird das Zwischenprodukt 10 durch eine Vernetzungsstation 20 geführt, um das Grundmaterial (vollständig) zu vernetzen. Die Vernetzung erfolgt beispielsweise durch thermische Behandlung oder auch durch UV-Behandlung etc. Das Zwischenprodukt 10 kann über Umlenkrollen 16 jeweils umgelenkt werden. Diese sind vorzugsweise nach der Vernetzungsstation 20 angeordnet, wie dies in Fig. 1C dargestellt ist. Bei der Ausgestaltung nach Fig. 1B ist gänzlich auf die Umlenkrollen 16 verzichtet.

Nach der Vernetzungsstation 20 durchläuft das Kabel 2 schließlich noch eine Reinigungsstufe 22. Im Ausführungsbeispiel ist dies ein Reinigungsbehälter, welcher mit einer Waschflüssigkeit gefüllt ist. Anschließend kann noch eine Trocknungsstufe angeschlossen sein, bevor dann das fertige Kabel 2 auf einer Trommel 24 aufgewickelt wird.

Ein derartig hergestelltes Kabel 2 ist beispielhaft in der FIG 2 dargestellt. Hieraus ist zu entnehmen, dass die einzelnen Feststoffpartikel 14 homogen verteilt in die Oberfläche 18 des Außenmantels 8 eingebettet sind, ohne in das Innenvolumen einzudringen. Die Oberfläche 18 ist dabei allgemein eine Außenfläche, die also mit äußeren Gegenständen in Berührung kommen kann. Die einzelnen Leitungsadern 6 weisen jeweils einen zentralen Leiter 26 auf, welcher von einer Leiterisolation 28 umgeben ist. Die Gruppe der Leitungsadern 6 ist unmittelbar vom Außenmantel 8 umgeben.

Wie in FIG 2 auch angedeutet ist, stehen die einzelnen Feststoffpartikel etwa schuppenartig aus der Oberfläche 18 hervor. Sie sind insbesondere als plättchenförmige Feststoffpartikel ausgebildet. Die Feststoffpartikel 14 sind ausschließlich in der Oberfläche 18 eingebettet und dringen maximal etwa bis zu ihrer Partikelgröße in die Oberfläche 18 ein.

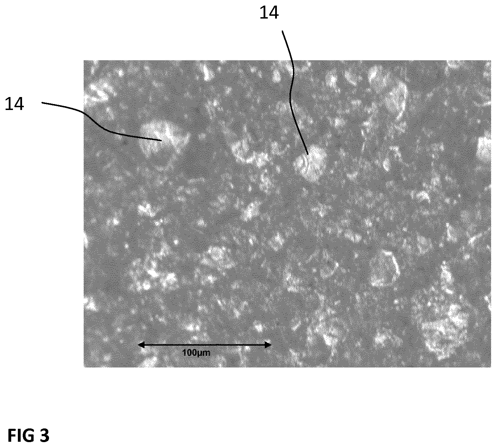

FIG 3 zeigt schließlich noch eine mikroskopische Vergrößerung einer derartig erzeugten Oberfläche, bei der die einzelnen Feststoffpartikel 14 durch die hellen Bereiche erkennbar sind. Die dunklen Bereiche zeigen demgegenüber die Matrix aus dem Silikon-Material, in der die Feststoffpartikel 14 eingebettet sind. Die größten Feststoffpartikel 14 weisen demnach eine Größe von maximal etwa 45µm auf.

Das hier beschriebene Verfahren ist nicht zwingend auf die Herstellung eines elektrischen Kabels mit einem Silikon-Kabelmantel begrenzt. Grundsätzlich lässt sich die grundlegende Idee, nämlich in die Oberfläche eines Silikon-Elements zur Verbesserung der Haptik und insbesondere zur Reduzierung des Reibungskoeffizienten Feststoffpartikel einzubringen, auch auf andere Produkte anwenden, welche an ihrer Oberfläche ein silikonartiges Grundmaterial aufweisen. Durch das hier beschriebene Verfahren wird in prozesstechnisch einfacher Weise ein Silikon-Produkt mit einer angenehmen Haptik und geringem Reibungskoeffizienten bereitgestellt. Als besonders vorteilhaft hat sich hierbei die Verwendung von Glimmerteilchen mit einer Korngröße im Bereich von 35µm bis 45µm herausgestellt. Derartige Silikon-Produkte eignen sich insbesondere für medizinische Anwendungen. Von besonderem Vorteil ist der Einsatz bei Kabeln, insbesondere für den medizinischen Bereich, da hierdurch die Handhabung verbessert und ein störendes Anhaften an Oberflächen oder auch auf der Haut vermieden ist.

### Bezugszeichen

- 2: Kabel
- 4: Extruder
- 6: Leitungsader
- 8: Außenmantel
- 10: Zwischenprodukt
- 12: Vorratsbehälter
- 14: Feststoffpartikel
- 16: Umlenkrolle
- 18: Oberfläche
- 20: Vernetzungsstation
- 22: Reinigungsstufe
- 24: Trommel
- 26: Leiter
- 28: Leiterisolation

## Patentansprüche

1. Verfahren zur Herstellung eines Elements, insbesondere eines Kabels (2), mit einem an der Oberfläche im Vergleich zu Silikon verminderten Reibungskoeffizienten, wobei das Element außenseitig ein silikonartiges Grundmaterial aufweist, das ein vernetzungsfähiger Kunststoff ist, welcher im vernetzten Endzustand einen zu Silikon vergleichbaren Reibungskoeffizienten aufweist, wie Silikone, Polyurethane, Polyamide, Polytetrafluorethylen (PTFE), Ethylen-Tetrafluorethylen (ETFE), Perfluorethylenpropylen (FEP), Perfluoralkoxy (PFA) sowie Copolymere hiervon, wobei das Grundmaterial in einem ersten Schritt zu einem zumindest teilweise unvernetzten Zwischenprodukt (10) ausgebildet wird, anschließend in einem zweiten Schritt Feststoffpartikel (14) in die Oberfläche (18) eingebracht werden und schließlich in einem dritten Schritt das unvernetzte Grundmaterial vernetzt wird
**dadurch gekennzeichnet,**
**dass** als Feststoffpartikel plättchenförmige Glimmerteilchen eingebracht werden wobei hierzu das unvernetzte Zwischenprodukt (10) im zweiten Schritt durch einen Vorratsbehälter (12) mit darin enthaltenen Feststoffpartikeln (14) geführt wird und der Vorratsbehälter (12) einer Schwingung ausgesetzt ist oder ein Wirbel bzw. ein Luftstrom im Vorratsbehälter (12) erzeugt wird, so dass die Feststoffpartikel mechanisch in die Oberfläche eingepresst werden und dass die Feststoffpartikel (14) über die Oberfläche verteilt sind, wobei zumindest ein Teil der Feststoffpartikel (14) schuppenartig aus der Oberfläche (18) hervorsteht.

2. Verfahren nach Anspruch 1, bei dem der Vorratsbehälter (12) als Rütteltrichter ausgebildet ist, durch den das Zwischenprodukt (10) geführt wird.

3. Verfahren nach einem der vorhergehenden Ansprüche, bei dem das Zwischenprodukt (10) in vertikaler Richtung durch den Vorratsbehälter (12) geführt wird.

4. Verfahren nach dem vorhergehenden Anspruch, bei dem das Zwischenprodukt (10) in vertikaler Richtung durch einen als Rütteltrichter ausgebildeten den Vorratsbehälter (12) geführt wird.

5. Verfahren nach einem der vorhergehenden Ansprüche, **dadurch gekennzeichnet, dass** das Zwischenprodukt (10) durch einen Extrusionsprozess ausgebildet wird und unmittelbar anschließend die Feststoffpartikel (14) eingebracht werden.

6. Verfahren nach einem der vorhergehenden Ansprüche,
**dadurch gekennzeichnet,**
**dass** nach dem dritten Schritt des Vemetzens überschüssige Feststoffpartikel (14) entfernt werden.

7. Verfahren nach einem der vorhergehenden Ansprüche,
**dadurch gekennzeichnet,**
**dass** die Feststoffpartikel (14) eine maximale Partikelgröße von 35µm - 55µm aufweisen.

8. Verfahren nach einem der vorhergehenden Ansprüche, bei dem folgende Schritte in einem kontinuierlichen Prozess unmittelbar aufeinanderfolgend ausgeführt werden
- Erzeugen des Zwischenprodukts (10) als kontinuierlicher Strang in einem Extrusionsprozess,
- Einbringen der Feststoffpartikel in die Oberfläche (18) des Zwischenprodukts (10)
- Vernetzen des silikonartigen Grundmaterials sowie bei Bedarf
- Reinigen von überschüssigen Feststoffpartikeln (14) und
- Aufwickeln des Strangs.

9. Verfahren nach einem der vorhergehenden Ansprüche,
**dadurch gekennzeichnet,**
**dass** das Element ein als Mantelleitung ausgebildetes elektrisches Kabel (2) ist mit zumindest einer Leitungsader (6), die von einem Außenmantel (8) aus dem silikonartigen Grundmaterial umgeben ist.

10. Verfahren nach Anspruch 1,
**dadurch gekennzeichnet, dass**
- der Vorratsbehälter (12) als Rütteltrichter ausgebildet ist, durch den das Zwischenprodukt (10) in vertikaler Richtung geführt wird,
- die Feststoffpartikel (14) eine maximale Partikelgröße von 35µm - 55µm aufweisen,
- das Zwischenprodukt (10) durch einen Extrusionsprozess ausgebildet wird und unmittelbar anschließend die Feststoffpartikel (14) eingebracht werden,
- nach dem dritten Schritt des Vernetzens überschüssige Feststoffpartikel (14) entfernt werden,
- das Element ein als Mantelleitung ausgebildetes elektrisches Kabel (2) ist mit zumindest einer Leitungsader (6), die von einem Außenmantel (8) aus dem silikonartigen Grundmaterial umgeben ist.

11. Element, insbesondere ein Kabel (2), das außenseitig ein silikonartiges Grundmaterial aufweist, das ein vernetzungsfähiger Kunststoff ist, welcher im vernetzten Endzustand einen zu Silikon vergleichbaren Reibungskoeffizienten aufweist, wie Silikone, Polyurethane, Polyamide, Polytetrafluorethylen (PTFE), Ethylen-Tetrafluorethylen (ETFE), Perfluorethylenpropylen (FEP), Perfluoralkoxy (PFA) sowie Copolymere hiervon, wobei zur Reduzierung eines Reibungskoeffizienten in der Oberfläche (18) des Grundmaterials Feststoffpartikel (14) eingebettet sind
**dadurch gekennzeichnet,**
**dass** als Feststoffpartikel plättchenförmige Glimmerteilchen in die Oberfläche mechanisch eingepresst sind und dass die Feststoffpartikel (14) über die Oberfläche verteilt sind, wobei zumindest ein Teil der Feststoffpartikel (14) schuppenartig aus der Oberfläche (18) hervorsteht.

12. Element nach Anspruch 11,
**dadurch gekennzeichnet,**
**dass** die Feststoffpartikel (14) eine maximale Partikelgröße von 35µm - 55µm aufweisen.

13. Element nach Anspruch 11 bis 12,
**dadurch gekennzeichnet,**
**dass** die Eindringtiefe der Feststoffpartikel (14) kleiner gleich ihrer Partikelgröße ist und das restliche Grundmaterial frei von Feststoffpartikeln (14) ist.

14. Element nach einem der Ansprüche 11 bis 13,
**dadurch gekennzeichnet,**
**dass** die Feststoffpartikel (14) ohne Vorzugsorientierung in der Oberfläche (18) eingebettet sind.

## Claims

1. Process for the production of an element, in particular a cable (2) with, at the surface, a coefficient of friction reduced in comparison with silicone, where the element comprises externally a silicone-like substrate material which is a crosslinkable plastic which in the crosslinked final state has a coefficient of friction comparable with that of silicone, examples being silicones, polyurethanes, polyamides, polytetrafluoroethylene (PTFE), ethylene-tetrafluoroethylene (ETFE), perfluorinated ethylene-propylene (FEP), perfluoroalkoxy (PFA) and also copolymers thereof, where the substrate material is configured in a first step to give an at least to some extent uncrosslinked intermediate product (10), then in a second step solid particles (14) are introduced into the surface (18) and finally in a third step the uncrosslinked substrate material is crosslinked, **characterized in that** mica particles in platelet form are introduced as solid particles, where for this purpose the uncrosslinked intermediate product (10) in the second step is passed through a storage container (12) with solid particles (14) present therein and the storage container (12) is subjected to oscillation or turbulence and/or an air stream is produced in the storage container (12) so that the solid particles are mechanically impressed into the surface and the solid particles (14) are distributed across the surface, where at least some of the solid particles (14) form scale-like projections from the surface (18).

2. Process according to Claim 1, where the storage container (12) is configured as vibratory hopper through which the intermediate product (10) is passed.

3. Process according to either of the preceding claims, where the intermediate product (10) is passed in vertical direction through the storage container (12).

4. Process according to the preceding claim, where the intermediate product (10) is passed in vertical direction through a storage container (12) configured as vibratory hopper.

5. Process according to any of the preceding claims, **characterized in that** the intermediate product (10) is configured by an extrusion process then immediately the solid particles (14) are introduced.

6. Process according to any of the preceding claims, **characterized in that** after the third step of the crosslinking, excess solid particles (14) are removed.

7. Process according to any of the preceding claims, **characterized in that** the maximal particle size of the solid particles (14) is 35 µm-55 µm.

8. Process according to any of the preceding claims, where the following steps are implemented in direct succession in a continuous process:
- production of the intermediate product (10) in the form of continuous strand in an extrusion process,
- introduction of the solid particles into the surface (18) of the intermediate product (10)
- crosslinking of the silicone-like substrate material, and also if necessary
- cleaning to remove excess solid particles (14) and
- wind-up of the strand.

9. Process according to any of the preceding claims, **characterized in that** the element is an electrical cable (2) configured as sheathed cable, with at least one conductor wire (6) surrounded by external sheathing (8) made of the silicone-like substrate material.

10. Process according to Claim 1, **characterized in that**
- the storage container (12) is configured as vibratory hopper through which the intermediate product (10) is passed in vertical direction,
- the maximal particle size of the solid particles (14) is 35 µm-55 µm,
- the intermediate product (10) is configured by an extrusion process and then immediately the solid particles (14) are introduced,
- after the third step of the crosslinking, excess particles (14) are removed,
- the element is an electrical cable (2) configured as sheathed cable, with at least one conductor wire (6) surrounded by external sheathing (8) made of the silicone-like substrate material.

11. Element, in particular a cable (2) externally comprising a silicone-like substrate material which is a crosslinkable plastic which in the crosslinked final state has a coefficient of friction comparable with that of silicone, examples being silicones, polyurethanes, polyamides, polytetrafluoroethylene (PTFE), ethylene-tetrafluoroethylene (ETFE), perfluorinated ethylene-propylene (FEP), perfluoroalkoxy (PFA), and also copolymers thereof, where solid particles (14) have been embedded in the surface (18) of the substrate material in order to reduce a coefficient of friction, **characterized in that** as solid particles platelet-shaped mica particles have been mechanically impressed into the surface and the solid particles (14) are distributed across the surface, where at least some of the solid particles (14) form scale-like projections from the surface (18).

12. Element according to Claim 11, **characterized in that** the maximal particle size of the solid particles (14) is 35 µm-55 µm.

13. Element according to Claims 11 to 12, **characterized in that** the penetration depth of the solid particles (14) is smaller than or equal to their particle size and the remainder of the substrate material is free from solid particles (14).

14. Element according to any of Claims 11 to 13, **characterized in that** the solid particles (14) have been embedded without preferential orientation in the surface (18).

## Revendications

1. Procédé de fabrication d'un élément, notamment d'un câble (2), présentant un coefficient de frottement sur la surface réduit en comparaison de la silicone, l'élément comprenant sur le côté extérieur un matériau de base de type silicone, qui est une matière plastique apte à la réticulation, qui présente à l'état final réticulé un coefficient de frottement comparable à la silicone, telle que les silicones, les polyuréthanes, les polyamides, le polytétrafluoroéthylène (PTFE), l'éthylène-tétrafluoroéthylène (ETFE), le perfluoroéthylène-propylène (FEP), le perfluoroalcoxy (PFA), ainsi que leurs copolymères, le matériau de base étant lors d'une première étape mis sous la forme d'un produit intermédiaire au moins partiellement non réticulé (10), puis, lors d'une deuxième étape, des particules solides (14) étant introduites dans la surface (18) et, enfin, lors d'une troisième étape, le matériau de base non réticulé étant réticulé, **caractérisé en ce que**
des particules de mica plaquettaires sont introduites en tant que particules solides, le produit intermédiaire non réticulé (10) étant pour cela acheminé lors de la deuxième étape au travers d'un contenant de stockage (12) dans lequel les particules solides (14) sont contenues, et le contenant de stockage (12) étant exposé à une vibration, ou un tourbillon ou un courant d'air étant généré dans le contenant de stockage (12), de telle sorte que les particules solides soient pressées mécaniquement dans la surface, et **en ce que** les particules solides (14) sont réparties sur la surface, au moins une partie des particules solides (14) dépassant de la surface (18) sous la forme d'écailles.

2. Procédé selon la revendication 1, dans lequel le contenant de stockage (12) est configuré sous la forme d'un entonnoir vibrant, au travers duquel le produit intermédiaire (10) est acheminé.

3. Procédé selon l'une quelconque des revendications précédentes, dans lequel le produit intermédiaire (10) est acheminé dans la direction verticale au travers du contenant de stockage (12).

4. Procédé selon la revendication précédente, dans lequel le produit intermédiaire (10) est acheminé dans la direction verticale au travers d'un contenant de stockage (12) configuré sous la forme d'un entonnoir vibrant.

5. Procédé selon l'une quelconque des revendications précédentes, **caractérisé en ce que** le produit intermédiaire (10) est formé par un processus d'extrusion, et les particules solides (14) sont introduites directement après.

6. Procédé selon l'une quelconque des revendications précédentes, **caractérisé en ce qu'**après la troisième étape de réticulation, des particules solides en excès (14) sont éliminées.

7. Procédé selon l'une quelconque des revendications précédentes, **caractérisé en ce que** les particules solides (14) présentent une taille de particule maximale de 35 µm à 55 µm.

8. Procédé selon l'une quelconque des revendications précédentes, dans lequel les étapes suivantes sont réalisées directement les unes après les autres dans le cadre d'un procédé continu :
- la formation du produit intermédiaire (10) sous la forme d'un boudin continu dans un processus d'extrusion,
- l'introduction des particules solides dans la surface (18) du produit intermédiaire (10),
- la réticulation du matériau de base de type silicone, et au besoin,
- l'élimination de particules solides en excès (14), et
- l'enroulement du boudin.

9. Procédé selon l'une quelconque des revendications précédentes, **caractérisé en ce que** l'élément est un câble électrique (2) configuré sous la forme d'un conducteur sous gaine, comprenant au moins un fil conducteur (6), qui est entouré par une gaine extérieure (8) en le matériau de base de type silicone.

10. Procédé selon la revendication 1, **caractérisé en ce que**
- le contenant de stockage (12) est configuré sous la forme d'un entonnoir vibrant, au travers duquel le produit intermédiaire (10) est acheminé dans la direction verticale,
- les particules solides (14) présentent une taille de particule maximale de 35 µm à 55 µm,
- le produit intermédiaire (10) est formé par un processus d'extrusion, et les particules solides (14) sont introduites directement après,
- après la troisième étape de réticulation, des particules solides en excès (14) sont éliminées,
- l'élément est un câble électrique (2) configuré sous la forme d'un conducteur sous gaine, comprenant au moins un fil conducteur (6), qui est entouré par une gaine extérieure (8) en le matériau de base de type silicone.

11. Élément, notamment câble (2), qui comprend sur le côté extérieur un matériau de base de type silicone, qui est une matière plastique apte à la réticulation, qui présente à l'état final réticulé un coefficient de frottement comparable à la silicone, telle que les silicones, les polyuréthanes, les polyamides, le polytétrafluoroéthylène (PTFE), l'éthylène-tétrafluoroéthylène (ETFE), le perfluoroéthylène-propylène (FEP), le perfluoroalcoxy (PFA), ainsi que leurs copolymères, des particules solides (14) étant incorporées dans la surface (18) du matériau de base pour la réduction d'un coefficient de frottement, **caractérisé en ce que**
des particules de mica plaquettaires sont pressées mécaniquement dans la surface en tant que particules solides, et **en ce que** les particules solides (14) sont réparties sur la surface, au moins une partie des particules solides (14) dépassant de la surface (18) sous la forme d'écailles.

12. Élément selon la revendication 11, **caractérisé en ce que** les particules solides (14) présentent une taille de particule maximale de 35 µm à 55 µm.

13. Élément selon les revendications 11 à 12, **caractérisé en ce que** la profondeur de pénétration des particules solides (14) est inférieure ou égale à leur taille de particule, et le matériau de base restant est exempt de particules solides (14).

14. Élément selon l'une quelconque des revendications 11 à 13, **caractérisé en ce que** les particules solides (14) sont incorporées dans la surface (18) sans orientation préférentielle.
